# EUROPEAN PATENT APPLICATION

(11) **EP 4 534 058 A1**
(43) Date of publication of application: **09.04.2025**
(21) Application number: 23810666.0
(22) Date of filing: 31.03.2023
(51) Int. Cl.: A61F 13/42, G01N 25/00, G01N 33/48

(54) **METABOLITE DETECTION METHOD AND NURSING ARTICLE**

(30) Priority: 27.05.2022 CN 202210589186
(71) Applicant: Shenda Chuangxin (Shenzhen) Technology Co., Ltd, Shenzhen, Guangdong 518051 (CN)
(72) Inventor: XIE, Jiquan, Shenzhen, Guangdong 518051 (CN); XIE, Junda, Shenzhen, Guangdong 518051 (CN)
(74) Representative: Leonard, Thomas Charles
(86) International application number: PCT/CN2023/085465
(87) International publication number: WO 2023/226591

(57) **Abstract**

A metabolite detection method and a nursing article. The metabolite detection method is applied to the nursing article. An absorption portion (101) of the nursing article is provided with a humidity sensing module for sensing a humidity value of a first position point set. The first position point set comprises at least one position point located on a metabolite diffusion path of the absorption portion (101). The metabolite detection method comprises: acquiring a humidity value of a first position point set, and determining growth stae information of a position point according to the humidity value, the growth state information being used for representing the growth condition of the humidity of the position point over time (S110); and determining a metabolite type according to the growth state information, the metabolite type comprising a liquid metabolite sand non-liquid metabolite (S120).

## Description

### TECHNICAL FIELD

The present invention relates to the field of nursing product detection technology, and particularly to a metabolite detection method and a nursing product.

### BACKGROUND

Diapers, disposable diapers, sanitary napkins, nursing pads, etc., are widely used by infants, women, and people with incontinence. The nursing product is made of flexible substrates such as PI, PET, nylon, and non-woven fabrics, etc. The absorption portion of the nursing product is usually in the shape of a long strip, which is configured to absorb or adhere to metabolites when worn to prevent leakage of metabolites.

However, the existing nursing product has a single function and cannot distinguish state types of the metabolites.

### SUMMARY

In view of this, it is necessary to provide a metabolite detection method and a nursing product capable of identifying the sate type of the metabolite.

A metabolite detection method is provided, which is applied to a nursing product, an absorption portion of the nursing product being provided with a humidity sensing module configured to sense humidity values of a first set of position points, the first set of position points including at least one position point located on a metabolite diffusion path of the absorption portion, the method includes:
acquiring the humidity values of the first set of position points, and determining growth state information of the position point according to the humidity values, the growth state information being configured to indicate a growth situation of a humidity at a position point over time;
determining a metabolite type according to the growth state information, the metabolite type including a liquid metabolite and a non-liquid metabolite.

In an embodiment, the growth state information includes a humidity growth rate, and the determining the metabolite type according to the growth state information includes:
when the humidity growth rate of the at least one position point is greater than a rate threshold value, determining the metabolite type as the liquid metabolite.

In an embodiment, the growth state information includes a maintenance duration in which a humidity value remains within a preset humidity range after increasing, the non-liquid metabolite includes a semi-solid metabolite, and the determining the metabolite type according to the growth state information includes:
when the maintenance duration of the at least one position point is greater than a first time threshold value, determining the metabolite type as the semi-solid metabolite; and/or
when the maintenance duration of any position point is less than a second time threshold value, determining the metabolite type as a liquid metabolite.

In an embodiment, the growth state information includes a maintenance duration in which a humidity value remains within a preset humidity range after increasing, the absorption portion of the nursing product is further provided with a temperature sensing module configured to sense temperature values of a second set of position points, the second set of position points includes at least one common position point which is a position point in the first set of position points, the non-liquid metabolite includes a semi-solid metabolite, and the method further includes:
acquiring the temperature values of the second set of position points;
when growth state information of the at least one common position point satisfies a preset condition and a temperature value at the common position point gradually decreases, determining the metabolite type as the semi-solid metabolite;
the preset condition includes that the humidity value remains within the preset humidity range after increasing.

In an embodiment, the absorption portion of the nursing product is further provided with a temperature sensing module configured to at least sense a temperature value at a first position point corresponding to a stool excretion site of a user, the first set of position points at least includes the first position point, the non-liquid metabolite includes a solid metabolite, and the method further includes:
acquiring a temperature value at the first position point;
when the temperature value at the first position point increases, and the humidity value at the first position point is less than the first humidity threshold value, determining the metabolite type as the solid metabolite.

In an embodiment, the absorption portion of the nursing product is further provided with a temperature sensing module configured to sense a temperature value of a second set of position points, the second set of position points include a plurality of position points on the absorption portion corresponding to excretion sites of a user when the nursing product is worn, and the method further includes:
acquiring temperature values of the second set of position points, and temperature diffusion information is determined according to the temperature values, the temperature diffusion information being configured to indicate an order in which the temperatures at the position points increase;
determining the metabolite type according to the temperature diffusion information.

In an embodiment, the second set of position points include a first position point corresponding to a stool excretion site, a second position point corresponding to a urination excretion site of a female user, and a third position point corresponding to a urination excretion site of a male user, a distance between the second position point and the first position point being smaller than a distance between the third position point and the first position point, and the determining the metabolite type according to the temperature diffusion information includes:
when the temperature diffusion information indicates that temperatures at the third position point, the second position point, and the first position point successively increase, or the temperatures at the second position point, the first position point, and the third position point successively increase, determining the metabolite type as the liquid metabolite.

In an embodiment, the determining the metabolite type according to the temperature diffusion information further includes:
when the temperature diffusion information indicates that the temperatures at the first position point, the second position point, and the third position point successively increase, determining the metabolite type as the non-liquid metabolite.

In an embodiment, when the metabolite type is the liquid metabolite, the method further includes:
determining a metabolic amount of liquid metabolites according to the humidity values;
when the metabolic amount exceeds a warning value, outputting warning information.

In an embodiment, the determining the metabolic amount of liquid metabolites according to the humidity values includes:
determining a target quantity according to the humidity values, the target quantity being the number of position points at which the humidity values are greater than a second humidity threshold value;
determining the metabolic amount according to the target quantity.

In an embodiment, the first set of position points at least includes a target position point, the target position point includes at least one of a second position point and a third position point, the second position point corresponds to a urination excretion site of a female user, the third position point corresponds to a urination excretion site of a male user, and the determining the metabolic amount of liquid metabolites according to the humidity values includes:
acquiring a time interval between a growth moment and an attenuation moment of a humidity value at the target position point, the growth moment referring to a moment when a growth rate of the humidity value at the target position point reaches a growth threshold value, and the attenuation moment refers to a moment when an attenuation rate of the humidity value at the target position point reaches an attenuation threshold value;
acquiring the metabolic amount according to the time interval and a preset excretion flow rate, the preset excretion flow rate referring to an excretion volume of the liquid metabolite per unit time.

In an embodiment, the absorption portion is further provided with a component detection module configured to detect metabolite component information of the at least one position point on the metabolite diffusion path of the absorption portion, and the method further includes:
acquiring the metabolite component information, and determining the metabolite type according to the metabolite component information.

A nursing product is provided, including:
a humidity sensing module, provided in an absorption portion of the nursing product and configured to sense humidity values of a first set of position points, the first set of position points including a plurality of position points located on a metabolite diffusion path of the absorption portion;
a detection module, configured to acquire the humidity values of the first set of position points, determine growth state information of the position points according to the humidity values, the growth state information being configured to indicate growth situations of humidities at the position points over time, and determine a metabolite type according to the growth state information, the metabolite type including a liquid metabolite and a non-liquid metabolite.

In an embodiment, the first set of position points includes at least two subsets of position points. a distance between a position point in a different subset of position points and a position point corresponding to a urination excretion site of a user falls into a different distance range, and the humidity sensing module includes:
a plurality of humidity sensitive units, respectively configured to detect the humidity values at position points in the subsets of position points in a one-to-one correspondence, humidity sensitive units are configured to detect the same subset of position points forming a humidity sensitive module, and the humidity sensitive units in the humidity sensitive module are connected to each other in parallel.

In an embodiment, the humidity sensing module includes:
a plurality of first humidity sensitive units, respectively provided at the position points in the first set of position points in a one-to-one correspondence, a sensitivity of each first humidity sensitive unit being inversely proportional to a distance between the first humidity sensitive unit and the position point corresponding to the urination excretion site of the user.

In an embodiment, the humidity sensing module further includes:
a plurality of groups of second humidity sensitive units, connected to the first humidity sensitive units in a one-to-one correspondence, a straight line where the same group of second humidity sensitive units and the corresponding first humidity sensitive unit are located being perpendicular to a straight line where each first humidity sensitive device is located.

In an embodiment, the nursing product further includes:
a temperature sensing module, provided in the absorption portion and configured to sense temperature values of a second set of position points, the second set of position points including a plurality of position points on the absorption portion corresponding to excretion sites of a user when the nursing product is worn;
the detection module is further configured to acquire the temperature values of the second set of position points, determine temperature diffusion information according to the temperature values, and determine the metabolite type according to the temperature diffusion information, wherein the temperature diffusion information is configured to indicate an order in which temperatures at various position points increase;
the temperature sensing module includes a plurality of temperature sensing units, which are respectively provided at the position points in the second set of position points in a one-to-one correspondence.

In an embodiment, the temperature sensing unit includes a plurality of thermosensitive devices connected to each other in series or in parallel.

In an embodiment, the absorption portion includes:
a first base layer, a sensing portion of the humidity sensing module being provided on a side of the first base layer adjacent to a human body;
a first wicking layer, covering the sensing portion of the humidity sensing module and configured to absorb and latch a liquid in a metabolite.

In an embodiment, the nursing product further includes:
a component detection module, provided in the absorption portion and configured to detect metabolite component information of the at least one position point on the metabolite diffusion path of the absorption portion;
the detection module is further configured to acquire the metabolite component information and determine the metabolite type according to the metabolite component information;
the component detection module includes a liquid phase detector, the absorption portion further includes:
a second base layer, a sensing portion of the liquid phase detector is provided on a side of the second base layer adjacent to the human body;
a second wicking layer, covering the sensing portion of the liquid phase detector, and configured to absorb and latch the liquid in the metabolite; and/or
the component detection module includes a gas phase detector, the absorption portion further includes:
a third base layer, a sensing portion of the gas phase detector is provided on a side of the third base layer adjacent to the human body;
a latching layer, covering the sensing portion of the gas phase detector and configured to latch the gas in the metabolite.

In above method, the humidity values of the first set of position points sensed by the humidity sensing module are obtained, and the growth state information of each position point in the first set of position points is determined according to each humidity value, thereby determining the metabolite type according to the growth state information. The method is simple and effective.

### BRIEF DESCRIPTION OF THE DRAWINGS

In order to more clearly illustrate the technical solution in the embodiments of the present invention or the conventional technology, accompanying drawings required for description of the embodiments or the conventional technology are briefly introduced below. Obviously, the drawings described below are merely some embodiments of the present invention, and thoses skilled in the art can obtain other drawings based on these drawings without any creative work.
FIG. 1 is a schematic structure diagram of a nursing product according to an embodiment.
FIG. 2 is a flow chart showing a metabolite detection method according to an embodiment.
FIG. 3 is a flow chart showing a metabolite detection method according to another embodiment.
FIG. 4 is a flow chart showing a metabolite detection method according to another embodiment.
FIG. 5 is a flow chart showing a metabolite detection method according to another embodiment.
FIG. 6 is a flow chart showing a metabolite detection method according to another embodiment.
FIG. 7 is a flow chart showing a metabolite detection method according to another embodiment.
FIG. 8 is a flow chart showing a metabolite detection method according to another embodiment.
FIG. 9 is a flow chart showing a metabolite detection method according to another embodiment.
FIG. 10 is a schematic diagram of a humidity variation curve of a semi-solid metabolite according to an embodiment.
FIG. 11 is a schematic diagram of a humidity variation curve of a liquid metabolite according to an embodiment.
FIG. 12 is a flow chart showing a metabolite detection method according to another embodiment.
FIG. 13 is a flow chart showing a metabolite detection method according to another embodiment.
FIG. 14 is a schematic structure diagram of a nursing product according to another embodiment.
FIG. 15 is a schematic diagram of a partial structure of an absorption portion according to an embodiment.
FIG. 16 is a schematic diagram of a partial structure of an absorption portion according to another embodiment.

### DETAILED DESCRIPTION

In order to facilitate the understanding of the present invention, the present invention will be described more comprehensively below with reference to the relevant accompanying drawings. Embodiments of the present invention are shown in the accompanying drawings. However, the present invention may be implemented in many different forms and is not limited to the embodiments described herein. Rather, these embodiments are provided to make the present invention more thorough and comprehensive.

Unless defined otherwise, all technical and scientific terms used herein have the same meaning as commonly understood by one of ordinary skill in the art to which the present invention belongs. The terms used herein in the specification of the present invention are only for the purpose of describing specific embodiments and are not intended to limit the present invention.

It should be appreciated that the terms "first", "second", etc., used in the present invention may be used for describing various elements, but these elements are not limited by these terms. These terms are only used for distinguishing a first element from another element. For example, a first resistor could be referred to as a second resistor, and, similarly, a second resistor could be referred to as a first resistor without departing from the scope of the present invention. The first resistor and the second resistor are both resistors, but they are not the same resistor.

It should be appreciated that the "connection" in the following embodiments should be understood as "electrical connection", "communication connection", etc., when the connected circuits, modules, units, etc., have electrical signals or data transmission with each other.

As used herein, the singular forms "a", "an" and "the" may include the plural forms as well, unless the context clearly indicates otherwise. It should also be appreciated that the terms "including/including" or "having" and the like specify the presence of the stated features, wholes, steps, operations, components, portions or combinations thereof, but do not exclude the possibility of the presence or addition of one or more other features, wholes, steps, operations, components, portions or combinations thereof. Meanwhile, the term "and/or" used in this specification includes any and all combinations of the relevant listed items.

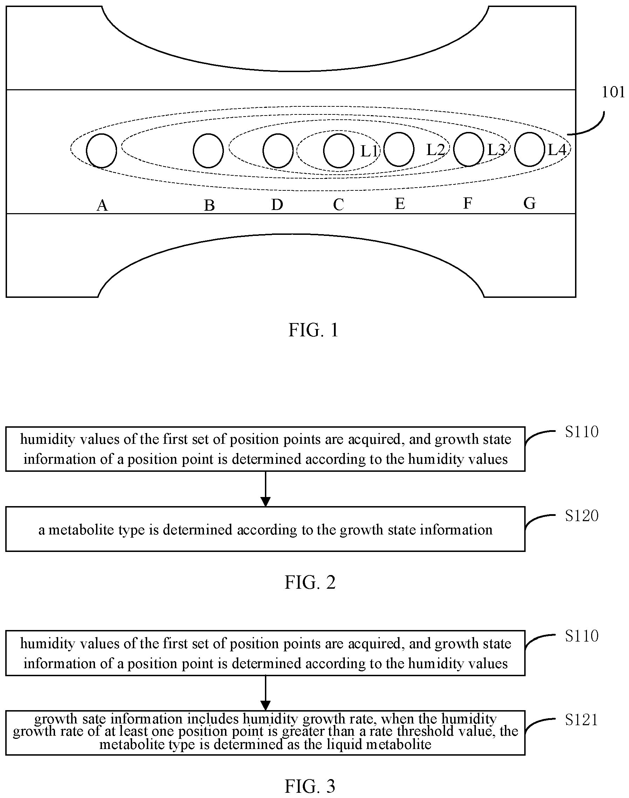

FIG. 1 is a schematic structure diagram of a nursing product according to an embodiment. The nursing product may include an absorption portion 101. As shown in FIG. 1, the absorption portion 101 is provided with a humidity sensing module configured to sense a humidity value of a first set of position points. The first set of position points may include at least one position point located on a metabolite diffusion path of the absorption portion 101. The metabolite detection method may include steps S110 to S120, as shown in FIG. 2.

Step S110: humidity values of the first set of position points are acquired, and growth state information of a position point is determined according to the humidity values, where the growth state information is configured to indicate a growth situation of a humidity at a position point over time.

It should be appreciated that the first set of position points may include one or more position points, which may be arranged at a position on the absorption portion 101 that can contact the metabolite. A plurality of position points are taken as an example, which may be located in a length direction of the absorption portion 101, such as position points A, B, C, D, E, and F in the figures. The humidity sensing module can continuously sense the humidity values at the position points, and the growth state information corresponding to the position points can be determined according to the humidity values at the position points. The growth state information may include at least one of a humidity growth rate, a situation where the humidity value remains within a preset humidity range after increasing, and a maintenance duration in which the humidity value remains within the preset humidity range after increasing.

Step S120: a metabolite type is determined according to the growth state information, and the metabolite type includes a liquid metabolite and a non-liquid metabolite.

The liquid metabolite may include urine or blood, and the non-liquid metabolite may include a solid metabolite and a semi-solid metabolite. The solid metabolite may be, for example, hard stool, and a semi-solid metabolite may be, for example, loose stool. The metabolite type may be determined according to the growth state information of one or more position points.

The absorption portion 101 may be manufactured by a flexible substrate, which may be PI, PET, nylon, non-woven fabric or other materials. A conductive layer is manufactured on the substrate, and the conductive layer may include a wire, a pad, a via, electrodes or electrical connection contacts, etc. A material of the conductive layer may include carbon, silver, copper, gold, or the like. A thickness of the substrate ranges from 0.05mm to 0.3mm. The substrate is in a strip shape with a length of 10mm to 300mm and a width of 3mm to 30mm.

In the embodiment of the present invention, the humidity values of the first set of position points sensed by the humidity sensing module are acquired, and the growth state information of each position point in the first set of position points is determined according to each humidity value, thereby determining the metabolite type according to the growth state information. The method is simple and effective.

In an embodiment, the growth state information may include a humidity growth rate, and the step of determining the metabolite type according to the growth state information may include step S121, as shown in FIG. 3.

Step S121: when the humidity growth rate of at least one position point is greater than a rate threshold value, the metabolite type is determined as the liquid metabolite.

The rate threshold value may be determined according to empirical values of the humidity growth rate when the liquid metabolite diffuses in the absorption portion 101. For example, the rate threshold value may be a minimum value of the humidity growth rate when the liquid metabolite diffuses in the absorption portion 101. It should be appreciated that the absorption rate of the absorption portion 101 for the liquid metabolite is faster, so that the humidity growth rate is also higher. By setting a rate threshold value, when a speed growth rate of a position point is greater than the rate threshold value, it indicates that the position point is in contact with the liquid metabolite.

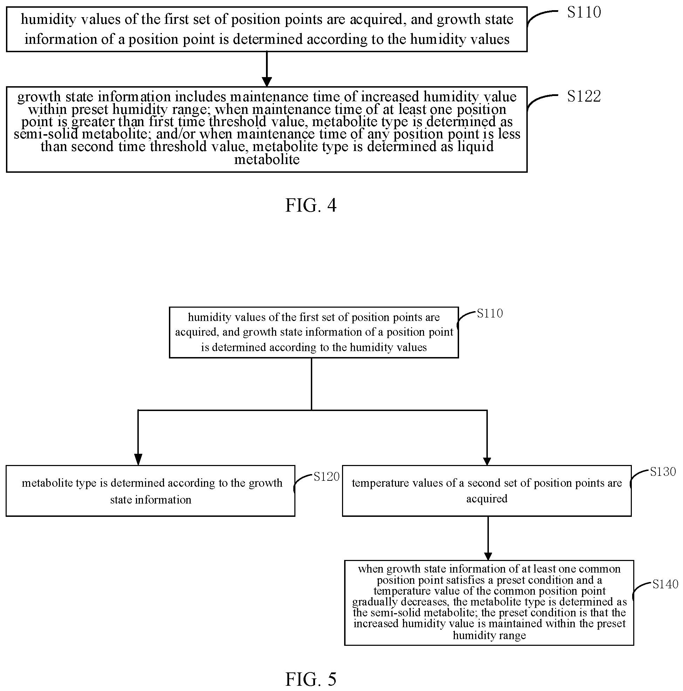

In an embodiment, the growth state information may include the maintenance duration in which the humidity value remains within the preset humidity range after increasing. The non-liquid metabolite may include a semi-solid metabolite. The step of determining the metabolite type according to the growth state information may further include step S122, as shown in FIG. 4.

Step S122: when the maintenance duration of at least one position point is greater than a first time threshold value, the metabolite type is determined as a semi-solid metabolite, and/or when the maintenance duration of any position point is less than a second time threshold value, the metabolite type is determined as a liquid metabolite.

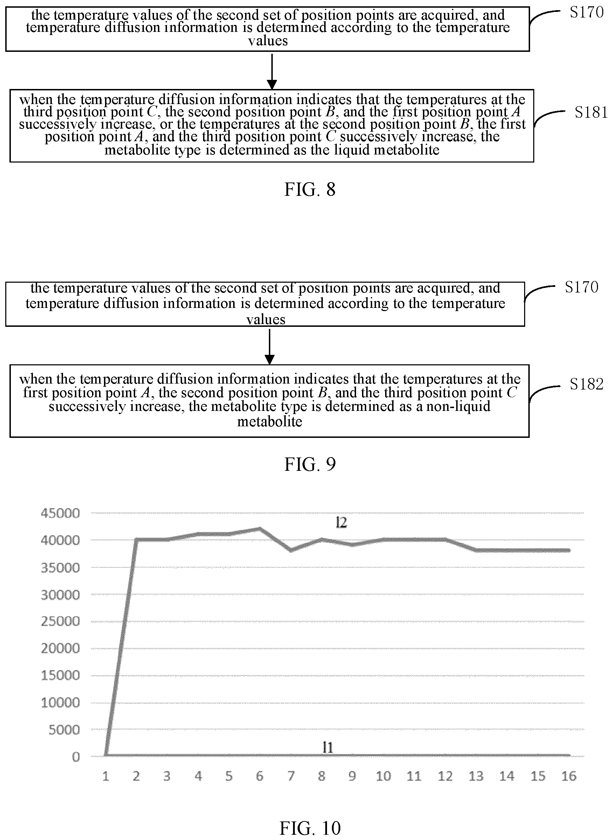

The semi-solid metabolite refers to stool with urine, for example, loose stool. It should be appreciated that the first time threshold value may be determined according to a usual duration of urination of a user, for example, the first time threshold value may be greater than or equal to a maximum urination time of the user. Since the liquid metabolite is quickly absorbed by the absorption portion 101 after being discharged, the corresponding humidity can only be maintained under the condition of continuous urination. Since the semi-solid metabolite contains a liquid such as urine, the urine in the semi-solid metabolite may not be quickly and completely absorbed by the absorption portion 101. Accordingly, when the semi-solid metabolite is excreted to a certain position point, the humidity at the position point may first growth sharply, and after reaching a peak value, the humidity may be maintained in a certain humidity range for a long time. In view of this, when the maintenance duration of at least one position point exceeds the first time threshold value, i.e., the normal urination time of the user, it indicates that the maintenance of humidity at the position point is not caused by the urination, but by the semi-solid metabolite. Thus, the metabolite type can be determined as the semi-solid metabolite in this case. The humidity of the semi-solid metabolite varies with time, as shown in FIG. 10, a curve 11 is a timevarying curve, and a curve l2 is a humidity value variation curve of the semi-solid metabolite. In an embodiment, the first time threshold value may be set according to empirical values.

Accordingly, the humidity at the position point may be maintained for a period of time.

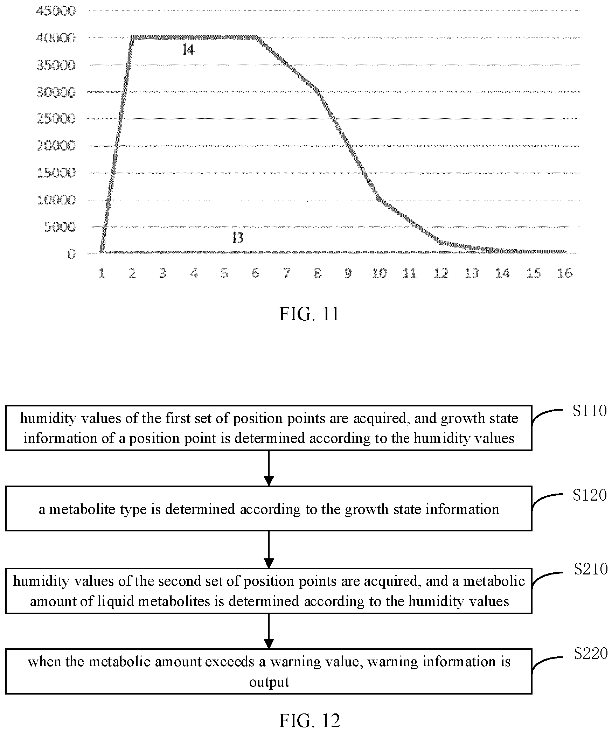

The liquid metabolite may be, for example, urine, blood, etc. The second time threshold value may also be determined according to a usual duration of urination of a user, for example, the second time threshold value may be less than or equal to the maximum urination time. It should be appreciated that liquid metabolite may be quickly absorbed by the absorption portion 101, so that the humidity value at the position point may increase sharply when the position point first comes to contact with the liquid metabolite. When there is no continuous liquid metabolite flowing through the position point, the humidity value at the position point may decrease rapidly. When the maintenance duration of at least one position point is less than the first time threshold value, that is, less than the normal duration of urination of the user, it may indicate that the maintenance of the humidity at the position point is caused by the urination, so that the metabolite type may be determined as the liquid metabolite at the moment. The humidity of the liquid metabolite varies with time. As shown in FIG. 11, a curve l3 is a time varying curve, and a curve l4 is a humidity value variation curve of the liquid metabolite. The first time threshold value may be equal to the second time threshold value.

In an embodiment, the growth state information may include the situation where the humidity value remains within the preset humidity range after increasing. The absorption portion 101 of the nursing product is further provided with a temperature sensing module configured to sense a temperature value of a second set of position points. The second set of position points may include at least one common position point which is a position point in the first set of position points. The non-liquid metabolite may include the semi-solid metabolite. The method may further include steps S130 to S140, as shown in FIG. 5.

Step S130: temperature values of a second set of position points are acquired.

It should be appreciated that the second set of position points may include one or more position points, each position point is located on the metabolite diffusion path of the absorption portion 101, and at least one position point among the position points is a position point in the first set of position points, that is, a common position point.

Step S140: when growth state information of at least one common position point satisfies a preset condition and a temperature value of the common position point gradually decreases, the metabolite type is determined as the semi-solid metabolite, and the preset condition includes that the humidity value remains within the preset humidity range after increasing.

It should be appreciated that since the liquid metabolite may be quickly absorbed by the absorption portion 101, and the humidity and temperature values may attenuate rapidly after increasing. But when the excretion is continuous, the humidity and temperature values can be maintained within a certain range. The semi-solid metabolite cannot be absorbed quickly, so that the humidity value thereof may be maintained within a certain range, and the temperature value thereof may gradually decrease. The position point sensed by the humidity sensing module may be the same as the position point sensed by the temperature sensing module, i.e., a common position point, and the humidity value and the temperature value at the common position point are acquired simultaneously. When the common position point is in contact with the metabolite, when the humidity value at the common position point increases and is then maintained within the preset humidity range, it indicates that the liquid metabolite are being continiuously excreted or that the metabolite is the semi-solid metabolite. Meanwhile, when the temperature value at the common position point gradually decreases, the situation of continuous excretion of the liquid metabolite can be excluded, and the metabolite can be determined as the semi-solid metabolite. When the humidity value at the common position point increases and is then maintained within the preset humidity range, and the temperature value gradually decreases, the metabolite type can be determined as the semi-solid metabolite.

The temperature sensing module may be provided with a temperature sensing unit 102 which may be manufactured by a thermosensitive material through printing, sputtering, evaporation and other processes, such as an NTC/PTC thermistor, a semiconductor temperature sensing chip, etc., and may be manufactured at a designated position by assembling, pasting, printing, deposition and other methods.

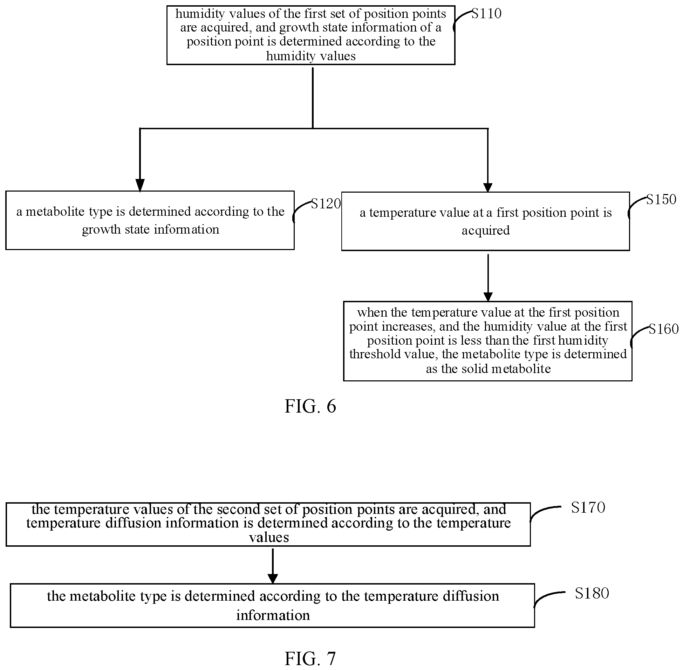

In an embodiment, the absorption portion 101 of the nursing product is further provided with a temperature sensing module configured to at least sense a temperature value at a first position point corresponding to the stool excretion site of the user. The first set of position points may at least include the first position point. The non-liquid metabolite may include a solid metabolite. The method may further include steps S150 to S160, as shown in FIG. 6.

Step S150: a temperature value at a first position point is acquired.

Step S160: when the temperature value at the first position point increases, and the humidity value at the first position point is less than the first humidity threshold value, the metabolite type is determined as the solid metabolite.

It should be appreciated that the first humidity threshold value may be less than or equal to the maximum humidity value of the absorption portion 101 when the solid metabolite is excreted to the absorption portion 101. When the temperature value at the first position point increases, it indicates that there exists a metabolite at the first position point. Meanwhile, when the humidity value at the first position point is less than the first humidity threshold value, it indicates that the metabolite is a solid metabolite. The solid metabolite may be, for example, dry stool.

In an embodiment, the absorption portion 101 of the nursing product is further provided with a temperature sensing module configured to sense temperature values of a second set of position points. The second set of position points may include a plurality of position points on the absorption portion 101 corresponding to various excretion sites of the user when the nursing product is worn. The method may further include steps S170 and S180, as shown in FIG. 7.

Step S170: the temperature values of the second set of position points are acquired, and temperature diffusion information is determined according to the temperature values; the temperature diffusion information is configured to indicate an order in which the temperatures at the position points increase.

It should be appreciated that the temperature sensing module continuously collects the temperature value at each position point, and when the metabolite is excreted to a position point, the temperature at the position point may increase. By acquiring the temperature value at each position point, and then the order in which the temperature at each position point increases can be determined according to the variation of each temperature value, that is, the order in which the metabolite fall into each position point. The increase of the temperature at the position point may refer to that the temperature value at the position point exceeds an initial value. In order to improve the detection accuracy, the increase of the temperature at the position point may also refer to that the temperature value at the position point exceeds a temperature threshold value.

Step S180: the metabolite type is determined according to the temperature diffusion information.

It should be appreciated that different metabolites may fall into different positions when excreted, and flow through different position points during the diffusion process. Accordingly, the order in which the metabolites fall into various positions after excreted can be acquired according to the temperature diffusion information. Based on the one-to-one correspondence between each position point and each excretion site of the human body, different metabolites flow through different position points in different orders after excreted, and the metabolite type can be determined accordingly.

There may exist a mapping relationship between the temperature diffusion information and the metabolite type, and the corresponding metabolite type can be obtained according to the temperature diffusion information. The metabolite type may include the liquid metabolite, such as urine, blood, etc., and may further include non-liquid metabolite, such as solid hard stool and pasty loose stool.

In an embodiment, as shown in FIG. 1, the second set of position points may include a first position point *A* corresponding to a stool excretion site, a second position point *B* corresponding to a urination excretion site of a female user, and a third position point *C* corresponding to a urination excretion site of a male user. A distance between the second position point *B* and the first position point *A* is smaller than a distance between the third position point *C* and the first position point *A.* The step of determining the metabolite type according to the temperature diffusion information may include step S181, as shown in FIG. 8.

Step S181: when the temperature diffusion information indicates that the temperatures at the third position point *C,* the second position point *B,* and the first position point *A* successively increase, or the temperatures at the second position point*B*, the first position point *A,* and the third position point *C* successively increase, the metabolite type is determined as the liquid metabolite.

It should be appreciated that the third position point *C* corresponds to a urination excretion site of a male user. When the user is a male, the urine may first flow through the third position point *C* when he urinates, causing the temperature at the third position point *C* to increase, and then diffuse to both ends. When diffusing toward the stool excretion site, the urine may flow through the second position point *B* and the first position point *A* in sequence, thereby causing the tempertures at the second position point *B* and the first position point *A* to increase successively. Therefore, when the temperatures at the third position point *C,* the second position point *B,* and the first position point A successively increase, it indicates that the male user excretes a liquid, and the metabolite may be urine.

The second position point *B* corresponds to a urination excretion site of a female user. When the user is a female, the metabolite may first flow through the second position point *B* when she urinates or in her menstrual period, causing the temperature at the second position point *B* to increase, and then diffuse to both ends. Since the distance between the first position point *A* and the second position point *B* is smaller than the distance between the third position point *C* and the second position point *B,* the first position point *A* may be in contact with the metabolite before the third position point *C,* and the temperature at the first position point *A* may increase before the temperature at the third position point *C.* Accordingly, when the temperatures at the second position point *B,* the first position point *A,* and the third position point *C* successively increase, it indicates that the female user excretes a liquid, and the metabolite may be urine or blood.

In an embodiment, the first set of position points may include a first position point *A* corresponding to a stool excretion site, a second position point *B* corresponding to a urination excretion site of a female user, and a third position point *C* corresponding to a urination excretion site of a male user. The distance between the second position point *B* and the first position point *A* is smaller than the distance between the third position point *C* and the first position point *A.* The step of determining the metabolite type based on the temperature diffusion information may further include step S182, as shown in FIG. 9.

Step S182: when the temperature diffusion information indicates that the temperatures at the first position point *A,* the second position point *B,* and the third position point *C* successively increase, the metabolite type is determined as a non-liquid metabolite.

It should be appreciated that the first position point *A* corresponds to the stool excretion site of the user. Regardless of whether the user is a male or a female, the metabolite may first flow through the first position point *A* during the excretion, causing the temperature at the first position point *A* to increase, and then diffuse to both ends. When the metabolite diffuses toward the urination excretion site of the user, the second position point *B* and the third position point are successively in contact with the metabolite, resulting in successive increases of the temperatures. Accordingly, when the temperatures at the first position point *A,* the second position point *B,* and the third position point *C* successively increase, it indicates that the user excretes the non-liquid metabolite, and the metabolite may be stool.

In the above embodiment, based on the flow condition of the metabolite on the nursing product during the excretion process of the user, the nursing product is provided with position points corresponding to the excretion sites of the human body, and then the metabolite type is determined according to the order in which the temperature at each position point in contact with the metabolite increase. The method is simple and the detection result is accurate.

In an embodiment, a diffusion rate of the metabolite may also be acquired according to temperature values, to determine that the metabolite is a solid metabolite or a semi-solid metabolite according to the diffusion rate.

In an embodiment, when the metabolite type is the liquid metabolite, the method may further include step S210 and step S220, as shown in FIG. 12.

Step S210: humidity values of the second set of position points are acquired, and a metabolic amount of liquid metabolites is determined according to the humidity values.

Step S220: when the metabolic amount exceeds a warning value, warning information is output.

It should be appreciated that in order to avoid excessive accumulation of the liquid metabolite in the nursing product, thereby affecting the subsequent absorption function of the nursing product and even affecting the health of the user, the metabolic amount of liquid metabolites can be further detected. When the metabolic amount exceeds the set warning value, the warning information is output to remind the user to replace the nursing product. The warning information may be a sound signal or display information.

In an embodiment, the step of determining the metabolic amount of liquid metabolites according to the humidity values may include step S211 and step S212.

Step S211: a target quantity is determined according to the humidity values; the target quantity is the number of position points at which the humidity values are greater than a second humidity threshold value.

Step S212: the metabolic amount is determined according to the target quantity.

It should be appreciated that when a humidity value at a position point is greater than the second humidity threshold value, it indicates that there exists a liquid metabolite at the position point. By reasonably setting the distances among the position points, the number of position points in contact with the liquid metabolite corresponds to the metabolic amount in a one-to-one correspondence, so that the corresponding metabolic amount can be obtained according to the number of position points.

As shown in FIG. 1, it is set that the first set of position points includes position points *A, B, C, D, E, F,* and *G*. Taking the user being a male as an example, penetration regions L1-L4 can be divided based on the position points, and areas of the penetration regions L1-L4 increase gradually. When only one position point is in contact with the liquid excrement, the liquid excrement is located in the region L1, and the metabolic amount corresponding to the region L1 may be equal to 20ml; when three position points are in contact with the liquid excrement, the liquid excrement is located in the region L2, and the metabolic amount corresponding to the region L2 may be equal to 40ml, and so on, the metabolic amount corresponding to the region L2 may be equal to 40ml, and the metabolic amount corresponding to the region L4 may be equal to 120ml. The corresponding metabolic amount can be determined according to the target quantity. The method is simple and can effectively measure the metabolic amount.

In an embodiment, the first set of position points may at least include a target position point, and the target position point may include at least one of a second position point *B* and a third position point *C.* The second position point corresponds to a urination excretion site of a female user, and the third position point corresponds to a urination excretion site of a male user. The step of determining the metabolic amount of liquid metabolites according to the humidity values may include steps S213 and S214.

Step S213: a time interval between a growth moment and an attenuation moment of the humidity value at the target position point is acquired; the growth moment refers to a moment when a growth rate of the humidity value at the target position point reaches a growth threshold value, and the attenuation moment refers to a moment when an attenuation rate of the humidity value at the target position point reaches an attenuation threshold value.

Step S214: the metabolic amount is acquired according to the time interval and a preset excretion flow rate; the preset excretion flow rate refers to an excretion volume of the liquid metabolite per unit time.

It should be appreciated that in order to determine the metabolic amount, the metabolic amount can be further determined by acquiring an excretion duration of the liquid metabolites in the excretion process of the user in combination with the excretion volume per unit time. The excretion duration refers to a time interval between a moment when the excretion of the user starts and a moment when the excretion of the user ends. The moment when the excretion starts can be represented by a moment when the humidity value corresponding to the urination excretion site of the user increases. Since the liquid excrement may be quickly absorbed after being excreted, if the excretion stops, the humidity value at the position point may decrease, so that the moment when the excretion ends can be represented by the attenuation moment when the humidity value corresponding to the urination excretion site of the user is attenuated. The humidity value at the position point at the growth moment may increase sharply, so that the growth moment can be determined according to whether the growth rate of the humidity value at the target position point reaches the growth threshold value. The humidity value at the position point at the attenuation moment may attenuate sharply, so that the attenuation moment can be determined according to whether the attenuation rate reaches the attenuation threshold value. The collected humidity values may have time stamps, so that the growth moment and the attenuation moment can be determined according to the humidity values with the time stamps.

In an embodiment, the absorption portion 101 is further provided with a component detection module configured to detect metabolite component information of at least one position point on the metabolite diffusion path of the absorption portion 101. The method may further include: the metabolite component information is acquired, and the metabolite type is determined according to the metabolite component information.

It should be appreciated that different metabolites contain different urea contents, so that it is possible to determine whether the metabolite is the liquid metabolite or the non-liquid metabolite according to the urea content in the metabolite.

The component detection module can be provided at a position corresponding to the excretion site of the user, and can also be provided at other positions that can be in contact with the metabolites during the excretion of the user.

In an embodiment, in order to improve the accuracy of the determination, the increase of the temperature at each position point may refer to that the temperature value at each position point exceeds a temperature threshold value.

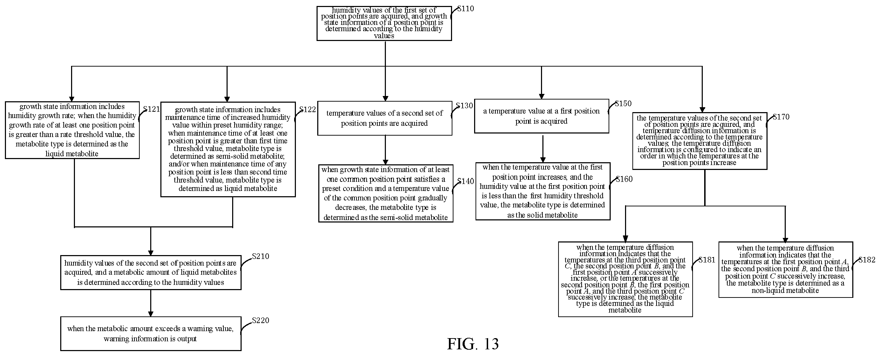

FIG. 13 is a flow chart of a metabolite detection method according to another embodiment, the specific steps of which have been specifically described in the above embodiments and will not be repeated here.

In an embodiment of the present invention, a nursing product is provided, which may include a humidity sensing module and a detection module. The humidity sensing module is provided in the absorption portion 101 of the nursing product, and is configured to sense humidity values of a first set of position points. The first set of position points includes a plurality of position points located on a metabolite diffusion path of the absorption portion 101. The detection module is configured to acquire the humidity values of the first set of position points, and determine growth state information of the position points according to the humidity values. The growth state information is configured to indicate a growth situation of the humidity at the position point over time. The detection module is further configured to determine a metabolite type according to the growth state information. The metabolite type includes a liquid metabolite and a non-liquid metabolite.

The principles and advantages of the nursing product in the embodiments are similar to those in the above-mentioned embodiments of the metabolite detection method, which are not described in detail here.

In an embodiment, the first set of position points may include at least two subsets of position points. A distance between a position point in a different subset of position points and a position point corresponding to a urination excretion site of a user falls into a different distance range. The humidity sensing module may include a plurality of humidity sensitive units. Humidity sensitive devices form at least two humidity sensitive modules, humidity sensitive units in the same humidity sensitive module are connected to each other in parallel; the humidity sensitive modules are respectively connected to the detection module, and are respectively configured to detect the humidity values at the position points in the subsets of position points in a one-to-one correspondence.

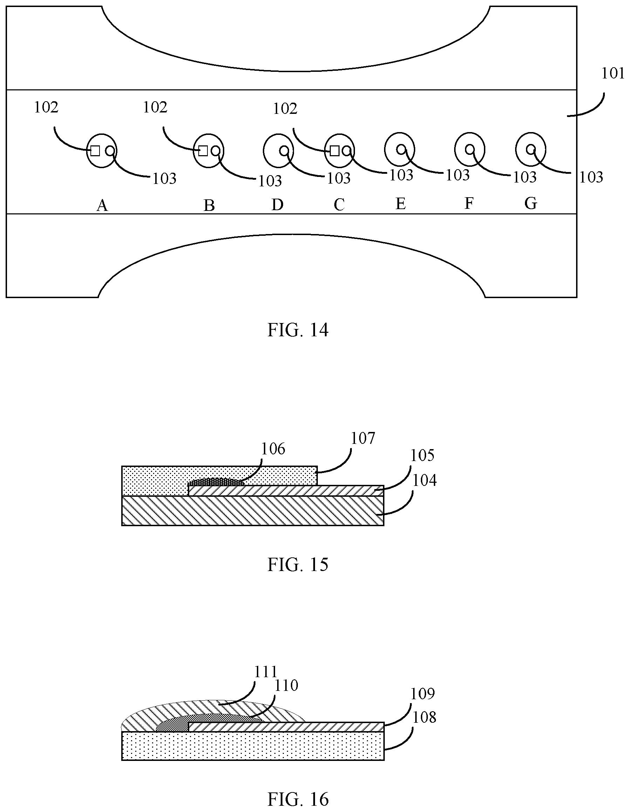

As shown in FIG. 14, it is set that a subset *B* of second position points may include position points *A, B, C, D, E, F,* and *G.* When the target quantity is determined according to the humidity values and then the metabolic amount of the liquid metabolite is determined, in order to reduce the complexity of the overall structue, at least two detection networks may be formed. The two detection networks are taken as an example, the position points D, C, E are closer to the excretion site and the humidity varies significantly, so that one detection network can be used. The position points A, B, F, G are far away from the excretion site and the variation of the humidity is not obvious, so that another detection network can be used. The humidity sensitive units 103 in the same detection network are connected to each other in parallel to reduce the complexity of the design structure. The humidity sensitive unit 103 may include one or more humidity sensitive devices. When humidity sensitive unit 103 includes a plurality of humidity sensitive devices, the humidity sensitive devices in the same humidity sensitive unit 103 may be connected to each other in series, in parallel, or in a combination of series and parallel. The humidity sensitive unit 103 may be a capacitive humidity sensitive unit 103 or a resistive humidity sensitive unit 103. When humidity sensitive unit is a capacitive humidity sensitive unit 103, a parallel connection mode is adopted; and when humidity sensitive unit is a resistive humidity sensitive unit 103, a series connection mode is adopted.

In an embodiment, a thermosensitive device and a humidity sensitive unit 103 located at the same position point can be integrated into a sensor unit to reduce the overall volume.

In an embodiment, the humidity sensing module may include a plurality of first humidity sensitive units 103, and the plurality of first humidity sensitive units 103 are respectively provided at the position points in the first set of position points in a one-to-one correspondence. A sensitivity of each first humidity sensitive unit 103 is inversely proportional to the distance between the first humidity sensitive unit 103 and the position point corresponding to the urination excretion site of the user.

It should be appreciated that, as shown in FIG. 14, the humidity sensing module may only include a row of first humidity sensitive units 103, which may be located in the middle of the absorption portion 101 to respectively detect the humidity value at each position point. When the target quantity is determined according to the humidity values and then the metabolic amount of the liquid metabolite is determined, in order to reduce costs and reduce the complexity of the overall structure, a first humidity sensitive unit 103 with a higher sensitivity may be adopted at a point approaching the urination excretion site, and a first humidity sensitive unit 103 with a lower sensitivity may be adopted at a point far from the urination excretion site. The first humidity sensitive unit 103 may include one or more humidity sensitive devices. When the first humidity sensitive unit includes a plurality of humidity sensitive devices, the humidity sensitive devices in the same humidity sensitive unit 103 may be connected to each other in series, in parallel, or in a combination of series and parallel. The sensitivity of the first humidity sensitive unit 103 can be regulated by changing the number of humidity sensitive devices.

In an embodiment, the humidity sensing module may further include a plurality of groups of second humidity sensitive units 103, which are connected to the first humidity sensitive units 103 in a one-to-one correspondence. A straight line where the same group of second humidity sensitive units 103 and the corresponding first humidity sensitive unit 103 are located is perpendicular to a straight line where each first humidity sensitive unit 103 is located.

It should be appreciated that, considering that the scenario where the nursing product may be worn sideways, a plurality of groups of second humidity sensitive units 103 may be provided to form an array of humidity sensitive units 103, so that the excretion can be accurately determined when any humidity unit detects the metabolites.

In an embodiment, the nursing product may further include a temperature sensing module, which is provided in the absorption portion 101 and is configured to sense the temperature values of the second set of position points. The second set of position points includes a plurality of position points on the absorption portion 101 corresponding to various excretion sites of the user when the nursing product is worn. The detection module is further configured to acquire the temperature values of the second set of position points, and determine temperature diffusion information according to the temperature values. The temperature diffusion information is configured to indicate an order in which temperatures at various position points increase. The detection module is further configured to determine the metabolite type according to the temperature diffusion information. The temperature sensing module includes a plurality of temperature sensing units 102, and each temperature sensing unit 102 is respectively provided at each position point in the first set of position points in a one-to-one correspondence.

As shown in FIG. 14, temperature sensing units 102 are provided at the first position point *A,* the second position point *B*, and the third position point *C* in the first set of position points to respectively detect the temperature value at each position point. A temperature sensing unit 102 may include one thermosensitive device or a plurality of thermosensitive devices.

In an embodiment, the temperature sensing unit 102 may include a plurality of thermosensitive devices, and the thermosensitive devices are connected to each other in series or in parallel.

It should be appreciated that in order to improve the detection accuracy of the temperature sensing unit 102, a plurality of thermosensitive devices may be provided to form the temperature sensing unit 102, to increase the detection range and provide a certain tolerance to the positions of the metabolites. Thermosensitive devices NTC made of a material with a negative temperature coefficient can be connected to each other in parallel, and thermosensitive devices PTC made of a material with a positive temperature coefficient can be connected to each other in series, to improve the sensitivity of the detection.

In an embodiment, the absorption portion 101 may include a first base layer and a first wicking layer. A sensing portion of the humidity sensing module is provided on a side of the first base layer adjacent to the human body; the first wicking layer covers the sensing portion of the humidity sensing module and is configured to absorb and latch liquid in a metabolite.

It should be appreciated that since water-absorbing resin particles in a core body of the absorption portion 101 of the nursing product have a very strong water-absorbing effect, in order to ensure enough moisture reaching a surface of the humidity sensitive unit, a first wicking layer can be manufactured on a surface of an electrode of the humidity sensitive unit 103 to latch moisture and ensure the accuracy of detection. The first wicking layer may include a water-absorbing material, such as water-absorbing resin, polyurethane, foam glue, etc.

In an embodiment, the nursing product may further include a component detection module, which is provided in the absorption portion 101 and is configured to detect metabolite component information of at least one position point on the metabolite diffusion path of the absorption portion 101. The detection module is further configured to acquire the metabolite component information and determine the metabolite type according to the metabolite component information. The component detection module may include a liquid phase detector and/or a gas phase detector.

When a liquid phase detector is included, as shown in FIG. 15, the absorption portion 101 may further include a second base layer 104 and a second wicking layer 107. A sensing portion 106 of the liquid phase detector is provided on a side of the second base layer 104 adjacent to the human body. The second wicking layer 107 covers the sensing portion 106 of the liquid phase detector, and is configured to absorb and latch the liquid in the metabolite. The reference sign 105 represents a connecting line of the liquid phase detector. A liquid phase substance to be detected may include but is not limited to at least one of pH value, glucose, dopamine, uric acid, ascorbic acid, galactose, ketone bodies, leukocytes, protein, occult blood, K+ ions, Na+ ions, iodine ions, calcium ions, iron ions, zinc ions, nitrites, etc.

It should be appreciated that liquid phase component analysis requires the establishment of a stable reaction zone to form a part of a wicking channel to ensure sufficient urine in the reaction zone within the detection time. Otherwise, the urine may be absorbed by the core layer of the diaper and cannot satisfy the requirement of a biochemical detection. The above-mentioned second wicking layer 107 provides a stable reaction zone, and the urine enters the reaction zone through the wicking action and is detected by a detector in the reaction zone. The second wicking layer 107 may include an interlayer and a hydrophilic layer from bottom to top. Furthermore, an inlet of the wicking channel for the liquid phase component analysis can also be implemented by drilling a hole in the base layer. The entire reaction zone can also be implemented by using a high molecular porous water-absorbing material.

When the component detection module includes a gas phase detector, as shown in FIG. 16, the absorption portion 101 may further include a third base layer 108 and a latching layer 111. A sensing portion 110 of the gas phase detector is provided on a side of the base layer adjacent to the human body. The latching layer 111 covers the sensing portion 110 of the gas phase detector and is configured to latch the gas in the metabolite. The reference sign 109 represents a connection line of the gas phase detector to transmit a sensing signal to the back end. A gas phase substance to be detected may include but is not limited to at least one of ammonia, hydrogen sulfide, hydrogen, skatole, etc. In addition, the latching layer 111 may be covered with a protective film layer to prevent the latching layer 111 from being physically scratched and damaged by foreign substances. The base layer is a carrier of the sensing portion and the connecting line.

The nursing product in the above embodiments may be a diaper, a nursing pad, a sanitary napkin, or other nursing products.

In the description of the specification, the description with reference to terms "some embodiments", "other embodiments", "ideal embodiments", etc., means that the specific features, structures, materials or characteristics described in conjunction with the embodiments or examples are included in at least one embodiment or example of the present invention. In the specification, the illustrative descriptions of the above terms do not definitely refer to the same embodiment or example.

The technical limitations in the above-described embodiments may be arbitrarily combined. To make the description concise, all possible combinations of the technical limitations in the above-described embodiments are not described. However, as long as there is no contradiction in combinations of these technical limitations, these combinations should be considered to be within the scope of the present invention.

The above-described embodiments merely express several implementation modes of the present invention, and the description thereof is relatively specific and detailed, but it should not be construed as limiting the scope of the present invention. It should be pointed out that, those skilled in the art can make several modifications and improvements without departing from the concept of the present invention, which all fall within the scope of protection of the present invention. Therefore, the protection scope of the present invention shall be subject to the appended claims.

## Claims

1. A metabolite detection method, applied to a nursing product, an absorption portion of the nursing product being provided with a humidity sensing module configured to sense humidity values of a first set of position points, the first set of position points comprising at least one position point located on a metabolite diffusion path of the absorption portion, the method comprising:
acquiring the humidity values of the first set of position points, and determining growth state information of the position point according to the humidity values, wherein the growth state information is configured to indicate a growth situation of a humidity at a position point over time;
determining a metabolite type according to the growth state information, wherein the metabolite type comprises a liquid metabolite and a non-liquid metabolite.

2. The metabolite detection method according to claim 1, wherein the growth state information comprises a humidity growth rate, and the determining the metabolite type according to the growth state information comprises:
when the humidity growth rate of the at least one position point is greater than a rate threshold value, determining the metabolite type as the liquid metabolite.

3. The metabolite detection method according to claim 1, wherein the growth state information comprises a maintenance duration in which a humidity value remtains within a preset humidity range after increasing, the non-liquid metabolite comprises a semi-solid metabolite, and the determining the metabolite type according to the growth state information comprises:
when the maintenance duration of the at least one position point is greater than a first time threshold value, determining the metabolite type as the semi-solid metabolite; and/or
when the maintenance duration of any position point is less than a second time threshold value, determining the metabolite type as a liquid metabolite.

4. The metabolite detection method according to claim 1, wherein the growth state information comprises a maintenance duration in which a humidity value remains within a preset humidity range after increasing, the absorption portion of the nursing product is further provided with a temperature sensing module configured to sense temperature values of a second set of position points, the second set of position points comprises at least one common position point which is a position point in the first set of position points, the non-liquid metabolite comprises a semi-solid metabolite, and the method further comprises:
acquiring the temperature values of the second set of position points;
when growth state information of the at least one common position point satisfies a preset condition and a temperature value at the common position point gradually decreases, determining the metabolite type as the semi-solid metabolite;
wherein the preset condition comprises that the humidity value remains within the preset humidity range after increasing.

5. The metabolite detection method according to claim 1, wherein the absorption portion of the nursing product is further provided with a temperature sensing module configured to at least sense a temperature value at a first position point corresponding to a stool excretion site of a user, the first set of position points at least comprises the first position point, the non-liquid metabolite comprises a solid metabolite, and the method further comprises:
acquiring a temperature value at the first position point;
when the temperature value at the first position point increases, and the humidity value at the first position point is less than the first humidity threshold value, determining the metabolite type as the solid metabolite.

6. The metabolite detection method according to claim 1, wherein the absorption portion of the nursing product is further provided with a temperature sensing module configured to sense a temperature value of a second set of position points, the second set of position points comprise a plurality of position points on the absorption portion corresponding to excretion sites of a user when the nursing product is worn, and the method further comprises:
acquiring temperature values of the second set of position points, and temperature diffusion information is determined according to the temperature values, wherein the temperature diffusion information is configured to indicate an order in which the temperatures at the position points increase;
determining the metabolite type according to the temperature diffusion information.

7. The metabolite detection method according to claim 6, wherein the second set of position points comprise a first position point corresponding to a stool excretion site, a second position point corresponding to a urination excretion site of a female user, and a third position point corresponding to a urination excretion site of a male user, wherein a distance between the second position point and the first position point is smaller than a distance between the third position point and the first position point, and the determining the metabolite type according to the temperature diffusion information comprises:
when the temperature diffusion information indicates that temperatures at the third position point, the second position point, and the first position point successively increase, or the temperatures at the second position point, the first position point, and the third position point successively increase, determining the metabolite type as the liquid metabolite.

8. The metabolite detection method according to claim 7, wherein the determining the metabolite type according to the temperature diffusion information further comprises:
when the temperature diffusion information indicates that the temperatures at the first position point, the second position point, and the third position point successively increase, determining the metabolite type as the non-liquid metabolite.

9. The metabolite detection method according to claim 1, wherein when the metabolite type is the liquid metabolite, the method further comprises:
determining a metabolic amount of liquid metabolites according to the humidity values;
when the metabolic amount exceeds a warning value, outputting warning information.

10. The metabolite detection method according to claim 9, wherein the determining the metabolic amount of liquid metabolites according to the humidity values comprises:
determining a target quantity according to the humidity values, wherein the target quantity is the number of position points at which the humidity values are greater than a second humidity threshold value;
determining the metabolic amount according to the target quantity.

11. The metabolite detection method according to claim 9, wherein the first set of position points at least comprises a target position point, the target position point comprises at least one of a second position point and a third position point, the second position point corresponds to a urination excretion site of a female user, the third position point corresponds to a urination excretion site of a male user, and the determining the metabolic amount of liquid metabolites according to the humidity values comprises:
acquiring a time interval between a growth moment and an attenuation moment of a humidity value at the target position point, wherein the growth moment refers to a moment when a growth rate of the humidity value at the target position point reaches a growth threshold value, and the attenuation moment refers to a moment when an attenuation rate of the humidity value at the target position point reaches an attenuation threshold value;
acquiring the metabolic amount according to the time interval and a preset excretion flow rate, wherein the preset excretion flow rate refers to an excretion volume of the liquid metabolite per unit time.

12. The metabolite detection method according to claim 1, wherein the absorption portion is further provided with a component detection module configured to detect metabolite component information of the at least one position point on the metabolite diffusion path of the absorption portion, and the method further comprises:
acquiring the metabolite component information, and determining the metabolite type according to the metabolite component information.

13. A nursing product, comprising:
a humidity sensing module, provided in an absorption portion of the nursing product and configured to sense humidity values of a first set of position points, wherein the first set of position points comprises a plurality of position points located on a metabolite diffusion path of the absorption portion;
a detection module, configured to acquire the humidity values of the first set of position points, determine growth state information of the position points according to the humidity values, wherein the growth state information is configured to indicate growth situations of humidities at the position points over time, and determine a metabolite type according to the growth state information, wherein the metabolite type comprises a liquid metabolite and a non-liquid metabolite.

14. The nursing product according to claim 13, wherein the first set of position points comprises at least two subsets of position points, a distance between a position point in a different subset of position points and a position point corresponding to a urination excretion site of a user falls into a different distance range, and the humidity sensing module comprises:
a plurality of humidity sensitive units, respectively configured to detect the humidity values at position points in the subsets of position points in a one-to-one correspondence, wherein humidity sensitive units are configured to detect the same subset of position points form a humidity sensitive module, and the humidity sensitive units in the humidity sensitive module are connected to each other in parallel.

15. The nursing product according to claim 13, wherein the humidity sensing module comprises:
a plurality of first humidity sensitive units, respectively provided at the position points in the first set of position points in a one-to-one correspondence, wherein a sensitivity of each first humidity sensitive unit is inversely proportional to a distance between the first humidity sensitive unit and the position point corresponding to the urination excretion site of the user.

16. The nursing product according to claim 15, wherein the humidity sensing module further comprises:
a plurality of groups of second humidity sensitive units, connected to the first humidity sensitive units in a one-to-one correspondence, wherein a straight line where the same group of second humidity sensitive units and the corresponding first humidity sensitive unit are located is perpendicular to a straight line where each first humidity sensitive device is located.

17. The nursing product according to claim 13, further comprising:
a temperature sensing module, provided in the absorption portion and configured to sense temperature values of a second set of position points, wherein the second set of position points comprises a plurality of position points on the absorption portion corresponding to excretion sites of a user when the nursing product is worn;
wherein the detection module is further configured to acquire the temperature values of the second set of position points, determine temperature diffusion information according to the temperature values, and determine the metabolite type according to the temperature diffusion information, wherein the temperature diffusion information is configured to indicate an order in which temperatures at various position points increase;
wherein the temperature sensing module comprises a plurality of temperature sensing units, which are respectively provided at the position points in the second set of position points in a one-to-one correspondence.

18. The nursing product according to claim 17, wherein the temperature sensing unit comprises a plurality of thermosensitive devices connected to each other in series or in parallel.

19. The nursing product according to claim 13, wherein the absorption portion comprises:
a first base layer, wherein a sensing portion of the humidity sensing module is provided on a side of the first base layer adjacent to a human body;
a first wicking layer, covering the sensing portion of the humidity sensing module and configured to absorb and latch a liquid in a metabolite.

20. The nursing product according to claim 13, further comprising:
a component detection module, provided in the absorption portion and configured to detect metabolite component information of the at least one position point on the metabolite diffusion path of the absorption portion;
wherein the detection module is further configured to acquire the metabolite component information and determine the metabolite type according to the metabolite component information;
wherein the component detection module comprises a liquid phase detector, the absorption portion further comprises:
a second base layer, wherein a sensing portion of the liquid phase detector is provided on a side of the second base layer adjacent to the human body;
a second wicking layer, covering the sensing portion of the liquid phase detector, and configured to absorb and latch the liquid in the metabolite; and/or
wherein the component detection module comprises a gas phase detector, the absorption portion further comprises:
a third base layer, a sensing portion of the gas phase detector is provided on a side of the third base layer adjacent to the human body;
a latching layer, covering the sensing portion of the gas phase detector and configured to latch the gas in the metabolite.
